(19) Europäisches Patentamt
European Patent Office
Office européen des brevets

(11) **EP 3 162 357 B1**

(12) **EUROPEAN PATENT SPECIFICATION**

(45) Date of publication and mention
of the grant of the patent:
**03.12.2025 Bulletin 2025/49**

(21) Application number: **14895569.3**

(22) Date of filing: **24.06.2014**

(51) International Patent Classification (IPC):
*A61K 8/9789* (2017.01)    *A61K 38/19* (2006.01)
*A61K 8/64* (2006.01)    *A61K 38/31* (2006.01)

(52) Cooperative Patent Classification (CPC):
**A61K 38/191; A61K 8/64; A61K 8/9789;
A61K 38/193; A61K 38/31**

(86) International application number:
**PCT/ES2014/070513**

(87) International publication number:
**WO 2015/197877 (30.12.2015 Gazette 2015/52)**

(54) **COSMETIC PRODUCT WITH LIPOSOMAL GROWTH FACTORS**

KOSMETISCHES PRODUKT MIT LIPOSOMALEN WACHSTUMSFAKTOREN

PRODUIT COSMÉTIQUE CONTENANT DES FACTEURS DE CROISSANCE LIPOSOMÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**03.05.2017 Bulletin 2017/18**

(73) Proprietor: **Dermopartners, S.L.
46138 Rafelbunyol - Valencia (ES)**

(72) Inventors:
• **SERRANO SANMIGUEL, Gabriel**
  **E-46138 Rafelbunyol - Valencia (ES)**
• **NAVARRO MOLINER, María**
  **E-46138 Rafelbunyol - Valencia (ES)**
• **TORRENS TOMAS, Ana**
  **E-46138 Rafelbunyol - Valencia (ES)**

(74) Representative: **Urizar Anasagasti, Jesus Maria
IPAMARK, S.L.
C/ Segre 27- 1°
28002 Madrid (ES)**

(56) References cited:
EP-A1- 1 941 901        WO-A1-2009/026949
WO-A2-2010/001417      WO-A2-2010/001417
US-A1- 2004 265 268    US-A1- 2004 265 268
US-A1- 2009 156 482

• BRAUND R. ET AL.: "The role of topical growth factors in chronic wounds.", CURRENT DRUG DELIVERY UNITED ARAB EMIRATES JUL 2007, vol. 4, no. 3, July 2007 (2007-07-01), pages 195 - 204, XP008184168, ISSN: 1567-2018

**Description**

## OBJECT OF THE INVENTION

The proposed invention relates to a novel cosmetic product with properties for repairing and rejuvenating the skin which includes in the composition thereof plant growth factors, in addition to antioxidants, anti-wrinkle peptides and stem cells and which is applicable to the sector of beauty, cosmetic and dermatology centers.

## BACKGROUND OF THE INVENTION

Many cosmetic preparations have been known hitherto which have effects for rejuvenating and repairing the skin, but preparations are not known that include liposomes with active ingredients such as high purity plant growth factors together with liposomated antioxidants, anti-wrinkle peptides and stem cells.

WO 2009/026949 discloses a skin care composition comprising a component of the present patent and then (in a list) varied growth factors (also granulocyte-macrophage colony stimulating factor), centella asiatica, quercetin and mixtures thereof. the active ingredients can be included in liposomes.

In EP1941901 an external preparation is shown, which comprises recombinant human growth hormone or recombinant human granulocyte macrophage-stimulating factor and pharmaceutically acceptable carrier (hGH and rhGM-CSF), in order to stimulate stem cells.

US 2004/265268 refers to a composition for skin repair comprising: a) at least one growth enhancer (EGF, FGF, IGF, GCSF, granulocyte macrophage colony stimulating factor (GMCSF), platelet derived growth factor (POGF), KGF, and tissue growth factor-[alpha] (TGF- [alpha]); b) at least one growth enhancer (cytokines, regulatory factors, angiogenic factors, adhesion proteins); c) at least one stimulator of cell growth enhancers (hyaluronic acid, sodium hyaluronate, ascorbic acid, sodium ascorbate, amphiregulin), wherein one option is to incorporate the active principles in liposomes.

WO2010/001417 describes a cosmetic and/or therapeutic composition comprising a recombinant non-plant, heterologous growth factor derived from a transgenic plant (stem cell factor, Granulocyte Macrophage Colony Stimulating Factor (GM- CSF)). Liposomes as pharmaceutical forms are suggested.

But none of these documents disclose the combination of a growth factor with an antioxidant (ergothioneine, quercetin, pterostilbene), anti-wrinkle peptides and a stem cell extract, wherein the first and second ingredient are packed in liposomes.

## DESCRIPTION OF THE INVENTION

The product which the invention proposes consists of a liposomated preparation in which the liposomes incorporate, as fundamental active ingredients, high purity plant growth factors and antioxidants, in addition to including, without liposomating, anti-wrinkle peptides and stem cells, according to claim 1.

The growth factors are natural proteins which stimulate cell growth, proliferation and differentiation. They play an important role in maintaining a structure of healthy skin, upon intervening in the communication between the epidermic and dermic cells by means of bonding to specific receptors on the surface of the cell.

It is well known that the growth factors play an important role for reversing the effects of aging of the skin, the topical application in individuals of growth factors has been demonstrated which acts at a local level and stimulates cell renovation, the reduction of wrinkles and the increase of collagen synthesis.

The main advantage of the product of the present invention is that it concerns high purity plant growth factors which, as they are incorporated in liposomes, their penetration through the skin and their stability is improved while avoiding their degradation like proteins that they are.

Among the plant growth factors included in the composition, there are the factors: HGH (human growth hormone), *Nicotiana benthamiana* Sh-Polypeptide-7, GM-CSF (Granulocyte Macrophage Colony-Stimulating Factor) *Nicotiana benthamiana* Sh-Polypeptide-45 and TGFb2 (Tumor growth factor beta 2) *Nicotiana benthamiana* Hexapeptide-40 Sh-Polypeptide-76.

*Nicotiana benthamiana* Sh-Polypeptide-7 is a single chain recombinant human peptide, produced in a transitory manner in the expression of Nicotiana benthamiana in plants. The starting gene is a copy synthesized from the human gene which codes for the growth hormone (GH) used as such or adapted to the host. It contains a maximum of 197 amino acids. The protein consists of the appropriate sequence of the 20 standard amino acids.

*Nicotiana benthamiana* Sh-Polypeptide-45 is a single chain of recombinant human peptide, produced in a transitory manner in the expression of Nicotiana benthamiana in plants. The starting gene is a synthetic copy of the human gene which codes for the factor for stimulating colonies of granulocytes and macrophages used as such or adapted for the production of the host. It contains a maximum of 127 amino acids which may contain disulfide bonds and/or glycosylation. The protein consists of the appropriate sequence of the 20 standard amino acids.

*Nicotiana benthamiana* Hexapeptide-40 sh-Polypeptide-76 contains two identical polypeptide chains joined by a single disulfide bond. They are produced in a transitory manner in the expression of Nicotiana benthamiana in plants. The starting gene is a synthetic copy of the human gene which codes for the transforming beta 2 growth factor used as such or adapted for the production of the host. It contains a maximum of 237 amino acids which may contain disulfide bonds and/or glycosylation. The protein consists of the appropriate sequence of the 20 standard amino acids.

In addition, among the liposomated antioxidants which are included in the composition there are antioxidants such as ergothioneine, quercetin and pterostilbene and among the anti-wrinkle peptides, also liposomated, there may be *Centella asiatica* extract, palmitoyl tripeptide-3 and caprooyl tetrapeptide-3.

Lastly, the product also incorporates as the active ingredient, without liposomating, extracts of stem cells of *Malus domestica.*

In the tests carried out, the data of which is stated below, it is checked that these growth factors in the form of liposomes maintain the same activity as the factor in free form, but their penetration through the skin and their stability is improved.

## PREFERRED EMBODIMENT OF THE INVENTION

As a preferred embodiment, a liposomated cosmetic product is proposed which has different cosmetic forms such as nutritive cream, solution, serum, emulsion, suspension, etc. in which the different components may be found in the case of the cream in the following proportions of active ingredients and of liposomes:

| INCI DESIGNATION | % | % LIPOSOMES |
|---|---|---|
| WATER | 50-75 | |
| CAPRYLIC/CAPRIC TRIGLYCERIDE | 1-5 | |
| SHEA BUTTER EXTRACT | 1-5 | |
| PROPANEDIOL | 1-5 | |
| SWEET ALMOND OIL | 1-5 | |
| DIMETICONE | 1-5 | |
| CETYL ALCOHOL | 1-5 | |
| GLYCERYL STEARATE | 1-5 | |
| LECITHIN | 1-5 | |
| CETEARYL ALCOHOL | 0.1-1 | |
| TOCOPHERYL ACETATE | 0.1-1 | |
| ALCOHOL | 0.1-1 | |
| PEG-75 STEARATE | 0.1-1 | |
| PHENOXYETHANOL | 0.1-1 | |
| POLYSORBATE 20 | 0.1-1 | |
| CETETH-20 | 0.1-1 | |
| STEARETH-20 | 0.1-1 | |
| XANTHAN GUM | 0.1-1 | |
| TRIETHANOLAMINE | 0.1-1 | |
| ACRYLATES/C10-30 ALKYL ACRYLATE CROSSPOL | 0.1-1 | |
| ETHYLHEXYLGLYCERIN | 0.1-1 | |
| SODIUM CHLORIDE | 0.1-1 | |
| MALUS DOMESTICA STEM CELL EXTRACT | 0-0.1 | |
| GLYCERIN | 0-0.1 | |
| DISODIUM EDTA | 0-0.1 | |

(continued)

| INCI DESIGNATION | % | % LIPOSOMES |
|---|---|---|
| PERFUME | 0-0.1 | |
| SODIUM CHOLATE | 0-0.1 | |
| PTEROSTILBENE | 0-0.1 | LIP.PTEROSTILBENE 1% |
| CENTELLA ASIATICA EXTRACT | 0-0.1 | LIP.CENTELLA ASIATICA EXTRACT 1.25% |
| PALMITOYL TRIPEPTIDE-3 | 0-0.1 | LIP.PALMITOYL TRIPEPTIDE -3 3.5% |
| CAPROOYL TETRAPEPTIDE-3 | 0-0.1 | LIP.CAPROOYL TETRAPEPTIDE -3 1.25% |
| QUERCETIN | up to 0.1 | LIP.QUERCETIN 1% |
| DEXTRAN | 0-0.1 | |
| TROMETHAMINE | 0-0.1 | |
| HYDROCHLORIC ACID | 0-0.1 | |
| ERGOTHIONEINE | up to 0.1 | LIP.ERGOTHIONEINE 1% |
| DIPOTASSIUM PHOSPHATE | 0-0.1 | |
| POTASSIUM PHOSPHATE | 0-0.1 | |
| NICOTIANA BETHANIANA SH-POLYPEPTIDE-7 | up to 0.1 | LIP.NICOTIANA BETHANIANA SH-POLYPEPTIDE-7 5% |
| NICOTIANA BENTHAMIANA HEXAPEPTIDE-40 SH-POLYPEPTIDE-76 | up to 0.1 | LIP. NICOTIANA BENTHAMIANA HEXAPEPTIDE-40 SH-POLYPEPTIDE-76 5% |
| NICOTIANA BETHAMIANA SH-POLYPEPTIDE-45 | up to 0.1 | LIP.NICOTIANA BETHAMIANA SH-POLYPEPTIDE-45 5% |

Another preferred embodiment is in the case of the serum where the different ingredients are found in the following proportions:

| INCI DESIGNATION | % | % LIPOSOMES |
|---|---|---|
| WATER | 75-100 | |
| GLYCERIN | 5-10 | |
| BIS-PEG-18 METHYL ETHER DIMETHYL SILANE | 1-5 | |
| PROPANEDIOL | 1-5 | |
| LECITHIN | 1-5 | |
| ALCOHOL | 1-5 | |
| HYDROGENATED CASTER OIL PEG-40 | 0.1-1 | |
| PHENOXYETHANOL | 0.1-1 | |
| POLYSORBATE 20 | 0.1-1 | |
| TOCOPHERYL ACETATE | 0.1-1 | |
| TRIETHANOLAMINE | 0.1-1 | |
| ACRYLATES/C10-30 ALKYL ACRYLATE CROSSPOL | 0.1-1 | |
| DISODIUM EDTA | 0.1-1 | |
| ETHYLHEXYLGLYCERIN | 0.1-1 | |
| SODIUM CHLORIDE | 0.1-1 | |

(continued)

| INCI DESIGNATION | % | % LIPOSOMES |
|---|---|---|
| PERFUME | 0.1-1 | |
| MALUS DOMESTICA STEM CELL EXTRACT | 0.1-1 | |
| SODIUM CHOLATE | 0.1-1 | |
| PTEROSTILBENE | 0.1-1 | LIP.PTEROSTILBENE 1% |
| XANTHAN GUM | 0.1-1 | |
| CENTELLA ASIATICA EXTRACT | 0.1-1 | LIP.CENTELLA ASIATICA EXTRACT 1.25% |
| PALMITOYL TRIPEPTIDE-3 | 0.1-1 | LIP.PALMITOYL TRIPEPTIDE - 3 3.5% |
| CAPROOYL TETRAPEPTIDE-3 | 0-0.1 | LIP.CAPROOYL TETRAPEPTIDE -3 1.25% |
| QUERCETIN | up to 0.1 | LIP.QUERCETIN 1% |
| DEXTRAN | 0-0.1 | |
| TROMETHAMINE | 0-0.1 | |
| HYDROCHLORIC ACID | 0-0.1 | |
| ERGOTHIONEINE | up to 0.1 | LIP.ERGOTHIONEINE 1% |
| DIPOTASSIUM PHOSPHATE | 0-0.1 | |
| POTASSIUM PHOSPHATE | 0-0.1 | |
| NICOTIANA BENTHAMIANA HEXAPEPTIDE-40 SH-POLYPEPTIDE-76 | up to 0.1 | LIP. NICOTIANA BENTHAMIANA HEXAPEPTIDE-40 SH-POLYPEPTIDE-76 8% |
| NICOTIANA BETHANIANA SH-POLYPEPTIDE-7 | up to 0.1 | LIP.NICOTIANA BETHANIANA SH-POLYPEPTIDE-7 5% |
| NICOTIANA BETHAMIANA SH-POLYPEPTIDE-45 | up to 0.1 | LIP.NICOTIANA BETHAMIANA SH-POLYPEPTIDE-45 5% |

## CLINICAL STUDIES AND DESCRIPTION OF THE DRAWINGS

ANALYSIS OF THE ACTIVITY OF THE FREE FACTOR VERSUS THE FACTOR ENCAPSULATED IN FIBROBLASTS OF SKIN AND HUMAN KERATINOCYTES

Factors analyzed:

- Factors GM-CSF (Granulocyte Macrophage Colony-Stimulating Factor), HGH (human growth hormone), TGFb2 (Tumor growth factor beta 2) and follistatin.
- Factors GM-CSF, HGH, TGFb2 and follistatin encapsulated in liposomes.

   GM-CSF and HGH $\rightarrow$ 50 mg/ml lipids, 400 ng/ml factor
   TGFb2 and follistatin $\rightarrow$ 50 mg/ml lipids, 200 ng/ml factor

Methods used:

- Cell proliferation
- Collagen elastin/synthesis by means of quantitative PCR
- Migration
- Anti-inflammatory effect by means of quantitative PCR

MTT CELL PROLIFERATION ASSAY

This assay is based on metabolic reduction of bromide 3-(4,5-dimethylthiazol-2-yl)-2,5-diphenyltetrazolium (MTT) carried out by the mitochondrial succinate dehydrogenase enzyme in a blue colored compound (formazan), allowing the mitochondrial functionality of the treated cells to be determined. This method has been very widely used to measure cell survival and proliferation.

The quantity of live cells is proportional to the quantity of formazan produced. This method was developed by Mosmann in 1983, modified in 1986 by Francois Denizot and Rita Lang.

Methodology

1. Using trypsin, separate the cells and re-suspend them in supplemented culture medium (10% of bovine fetal serum and antibiotic).

2. Determine the number of cells required per well using a Neubauer camera (HaCaT: 5,000 cells/96 well, 30,000 cells/24 well; HSF: 4,000 cells/96 well, 30,000 cells/24 well.

3. Incubate at 37°C and 5% of $CO_2$ for 24 hours to allow for the adherence thereof.

4. Prepare the mixtures of free and liposomated factor at the indicated concentrations, remove the medium and add the mixtures.

5. Incubate at 37°C and 5% of $CO_2$ for 72 hours.

6. Add MTT (1.9 mg/ml) in PBS solution (25 $\mu$l total) over the medium

7. Incubate for 3 hours at 37°C to allow for the formation of formazan crystals.

8. Then remove the supernatant and add 100 $\mu$l of DMSO.

9. Incubate for 30 minutes at 37°C to allow for the formazan crystals to dissolve

10. The optical density reading (OD) is carried out in a spectrophotometer at a wavelength of 570 nm.

The viability percentage is obtained in the following manner:

$$\% \text{ viability} - \text{DO cells treated x 100/ DO control cells}$$

WOUND HEALING ASSAY (CELL MIGRATION)

The objective of the wound healing assay is the study of cell migration. It is based on observing the behavior of a confluent cell monolayer on which a hole or wound has been previously made. The cells at the border of the hole move towards the opening until new cell-cell contacts are established, thus closing the wound.

The basic steps involve creating the wound or cell free area on the cell monolayer, the capture of images periodically during the experiment and the comparison of all the images to determine the velocity of cell migration.

Methodology

1. Cultivate the cells until confluence (100% approx.) in plates of 6 wells (HaCaT: 500,000 cells/6 well; HSF: 500,000 cells/6 well)

2. Incubate at 37°C and 5% of $CO_2$ for 24 hours.

3. Scaling of the wounds (2 wounds per horizontal trajectory well using yellow sterile points (P200). They are dragged along the monolayer with an angle of inclination of 30 degrees (not perpendicular to the plate) and guided with a previously marked section. Mark three points for taking measurements.

4. Leave to rest for 15 minutes before adding the treatment.

5. Prepare the mixtures of free and liposomated factors at a concentration of 1 ng/ml, the medium is removed and add the mixtures.

6. Incubate at 37°C and 5% of $CO_2$ for 72 hours.

7. Take measurements at the times 0, 24, 48 and 72 hours.

The percentage of migration is obtained in the following manner:

% migration = (time measured 0-time measured X / time measured 0) * 100

## GENE EXPRESSION ANALYSIS BY MEANS OF PCR IN REAL TIME. ELASTIN EXPRESSION ASSAY AND PROINFLAMMATORY CYTOKINES

PCR in real time or quantitative PCR is a variation of standard PCR used for quantifying DNA or messenger RNA (mRNA) of a sample. Using specific sequence primers, it is possible to determine the number of copies or the relative quantity of a determined DNA or RNA sequence.

When PCR in real time is combined with a retro-transcription or RT reaction (RTPCR), the mRNA quantity of a sample may be determined by means of a relative quantification. Said quantification is termed relative if the relative quantity or ratio of mRNA of a specific gene is compared with respect to the mRNA quantity of a constitutive gene (endogenous control, GAPDH in this case) among different samples. This is what is termed as normalization of the specific gene expression or normalizing with respect to the different total concentration of RNA of the samples since if the quantity of endogenous control varies it is due to changes in the total RNA quantity used in the synthesis of cDNA, not to changes in its expression. For the quantification, the quantity of amplicon produced is measured in each cycle of PCR. The quantification of the product is produced by means of the addition of fluorophores which join to the amplicon in a quantitative manner such that the greater the product, the greater the fluorescence that will be emitted.

The quantification method which has been used is ΔΔCt in which the Cts of the tested gene and reference gene (ΔCt) are compared directly in each sample and subsequently the (ΔCt) of the experimental samples are compared with respect to the control sample, in order to apply said method it is necessary for the efficiencies of both genes to be similar.

Methodology

1. Using trypsin, separate the cells and re-suspend them in supplemented culture medium (10% of bovine fetal serum and antibiotic).

2. Determine the number of cells required per well using a Neubauer camera (HaCaT: 9,000 cells/96 well; HSF: 9,000 cells/96 well)

3. Incubate at 37°C and 5% of $CO_2$ for 24 hours to allow for the adherence thereof.

4. Prepare the mixtures of free and liposomated factor at the indicated concentrations, remove the medium and add the mixtures.

5. Incubate at 37°C and 5% of $CO_2$ for 72 hours.

6. RNA extraction using the kit: E.Z.N.A.® Total RNA (Omega Bio-Tek, Inc., United States) following the manufacturer instructions. The isolated RNA was kept at -80°C until its use. A microgram of RNA, estimated by the spectrophotometry NanoDrop 200 c (Thermo FisherScientific Inc.) was used for obtaining cDNA using M-MLV reverse transcriptase (Invitrogen) according to the traditional procedures.

7. The PCRq assays were carried out using the ABI PRISM 7300 system (Applied Biosystems, NJ, USA) and SYBR green. The reactions were carried out in a volume of 20 μl in which there was 2 μl of cDNA, 900 nm of each primer, 200 nm of probe and 10 μl of the TaqMan universal mix master (Applied Biosystems) or SYBR green. The conditions of the cycles were 50°C for 2 minutes and 95°C for 10 mins, followed by 40 cycles at 95°C for 15 seconds and 60°C for 1 minute. The expression was analyzed by the method of 2-DCt where $\Delta$Ct is determined by subtracting the value of $\Delta$Ct from the GAPDH gene, used as the endogenous control, at the value of the objective Ct.

RESULTS

## 1. CELL PROLIFERATION

### 1.1 CELL PROLIFERATION WITH HGH

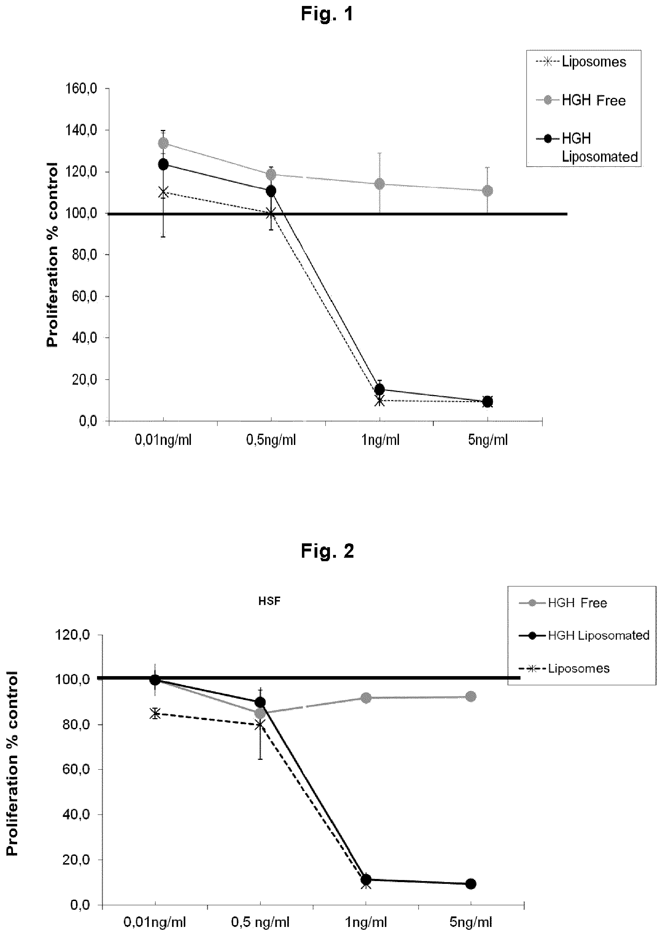

In Figure 1, it may be observed that in human keratinocytes, both the free and the liposomated factor generate cell proliferation of 20-40% above the untreated cells (which indicate 100% proliferation) at low concentrations (0.01 ng/ml - 0.5 ng/ml). However, at concentrations greater than 1 ng/ml, a high decrease of the cell viability is observed with the liposomated factor, but not with the free factor which maintains the cell proliferation around 20% above the untreated cells. In addition, the liposomes do not induce proliferation at low concentrations (0.01 ng/ml - 0.5 ng/ml), although they do induce a high decrease of the cell viability at concentrations greater than or equal to 1 ng/ml.

In addition in Figure 2, it may be observed that in human fibroblasts, the free factor does not generate proliferation, being maintained around 100%. However, at concentrations greater than 1 ng/ml, a high decrease of the cell viability is observed with the liposomated factor, but not with the free factor which is maintained around 100%. In addition, the liposomes induce a decrease of the cell viability (20%) at low concentrations (0.01 ng/ml - 05 ng/ml) and a high decrease of the cell viability at concentrations greater than or equal to 1 ng/ml.

### 1.2 CELL PROLIFERATION WITH GM-CSF

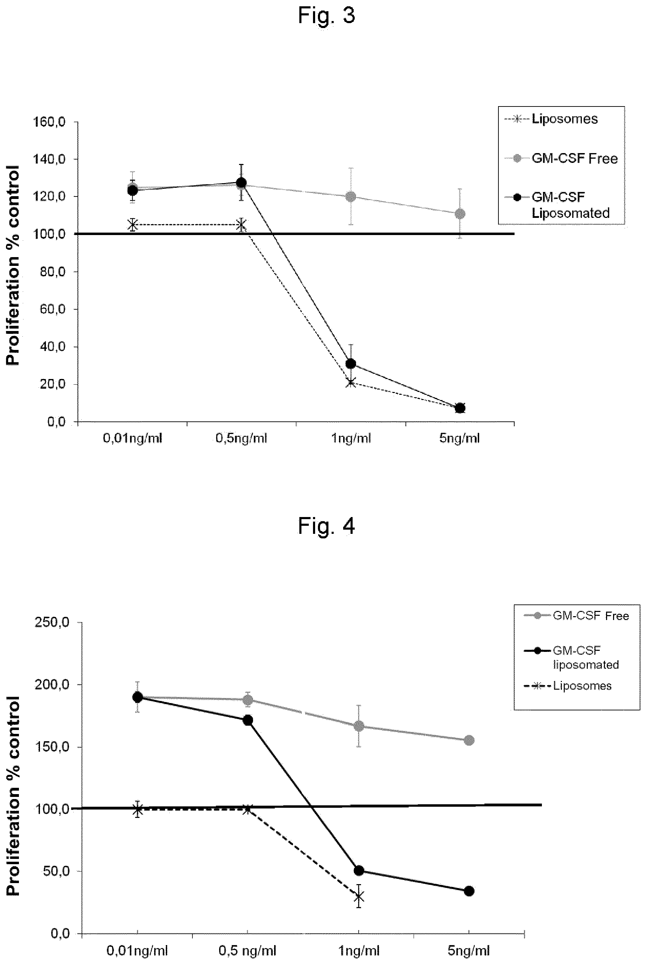

In Figure 3, it may be observed that in human keratinocytes, both the free and the liposomated factor generate cell proliferation of 20% above the untreated cells (which indicate 100% proliferation) at low concentrations (0.01 ng/ml - 0.5 ng/ml). However, at concentrations greater than 1 ng/ml, a high decrease of the cell viability is observed with the liposomated factor, but not with the free factor which maintains the cell proliferation around 20% above the untreated cells. In addition, the liposomes do not induce proliferation at low concentrations (0.01 ng/ml - 0.5 ng/ml), although they do induce a high decrease of the cell viability at concentrations greater than or equal to 1 ng/ml.

In addition in Figure 4, it may be observed that in human fibroblasts, the free factor and the liposomated factor generate cell proliferation of 100% above the untreated cells (which indicate 100% proliferation) at low concentrations (0.01 ng/ml - 0.5 ng/ml). However, at concentrations greater than 1 ng/ml, a decrease of the cell viability of 50-60% is observed with the liposomated factor, but not with the free factor which maintains cell proliferation around 60% above the untreated cells. In addition, the liposomes do not induce proliferation at low concentrations (0.01 ng/ml - 0.5 ng/ml), although they do induce a decrease of the cell viability of 50-60% at concentrations greater than or equal to 1 ng/ml.

### 1.3 CELL PROLIFERATION WITH FOLLISTATIN

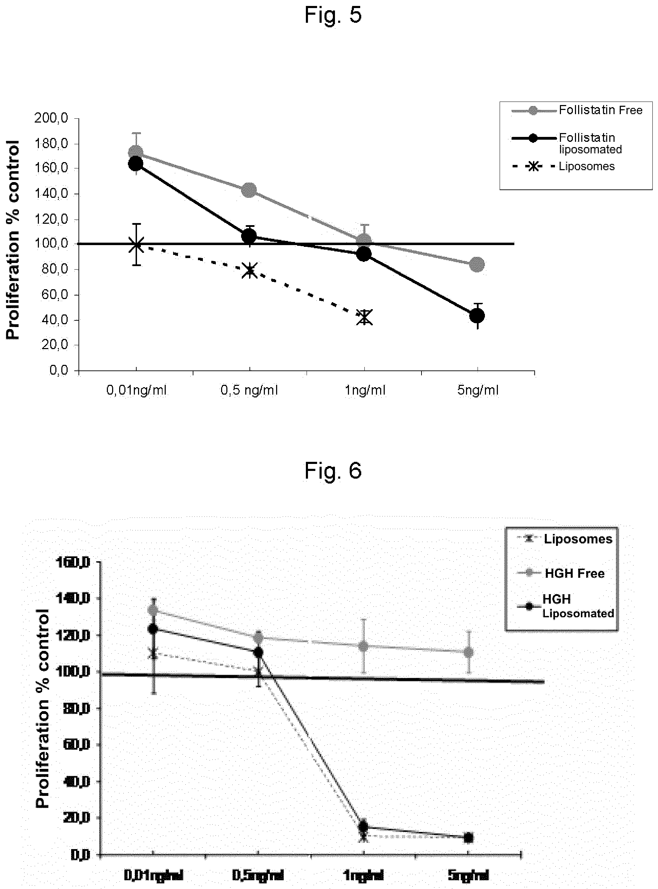

In Figure 5, it may be observed that in human fibroblasts, both the free factor and the liposomated factor generate cell proliferation of 60-70% above the untreated cells (which indicate 100% proliferation) at low concentrations (0.01 ng/ml). However, at a concentration of 0.5 ng/ml, the free factor generates a proliferation of 40%, while the liposomated factor does not generate proliferation. In addition, at concentrations greater than 1 ng/ml, a decrease of the cell viability of 50% is observed with the liposomated factor, but not with the free factor which maintains cell proliferation around the control. In addition, the liposomes do not induce proliferation at low concentrations (0.01 ng/ml - 05 ng/ml), although they do induce a decrease of the cell viability at concentrations greater than 0.5 ng/ml.

## 2. CELL MIGRATION (WOUND HEALING)

### 2.1 CELL MIGRATION WITH HGH

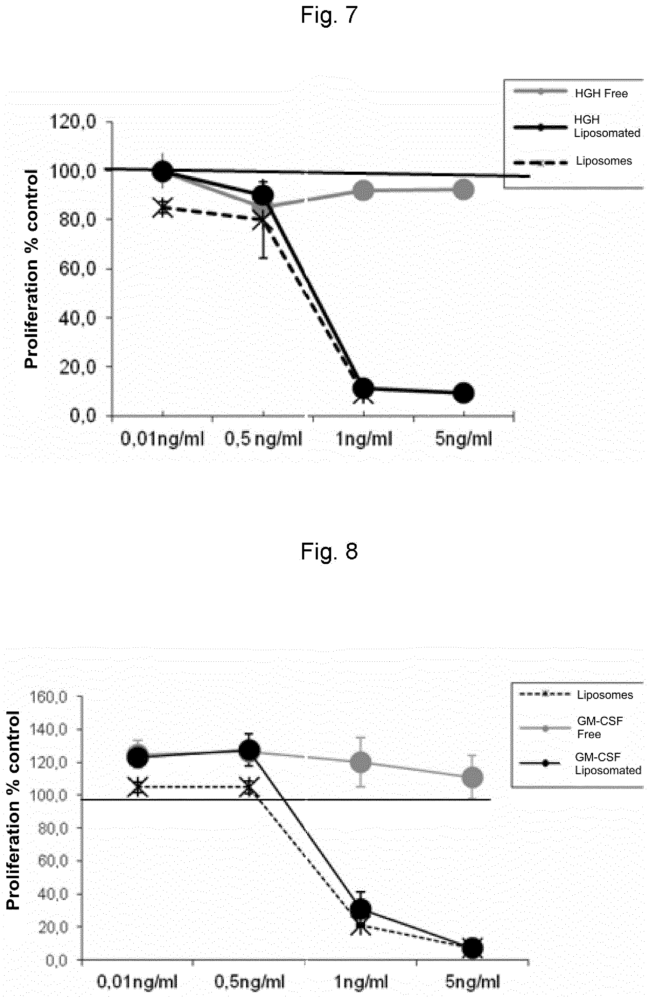

In Figure 6, a rapid repair velocity of the wound is observed with the free factor in human keratinocytes since at 24 hours of treatment, the wound had closed 40% more quickly than in the control cells and at 48 hours it was already completely closed. However, it is observed that the liposomated factor makes it difficult to close the wound, it being closed more slowly than in the control cells. However, in Figure 7, it is observed that in human fibroblasts there is hardly any difference seen in the repair velocity of the wound among the cells treated with free factor and the control cells.

2.2. CELL MIGRATION WITH GM-CSF

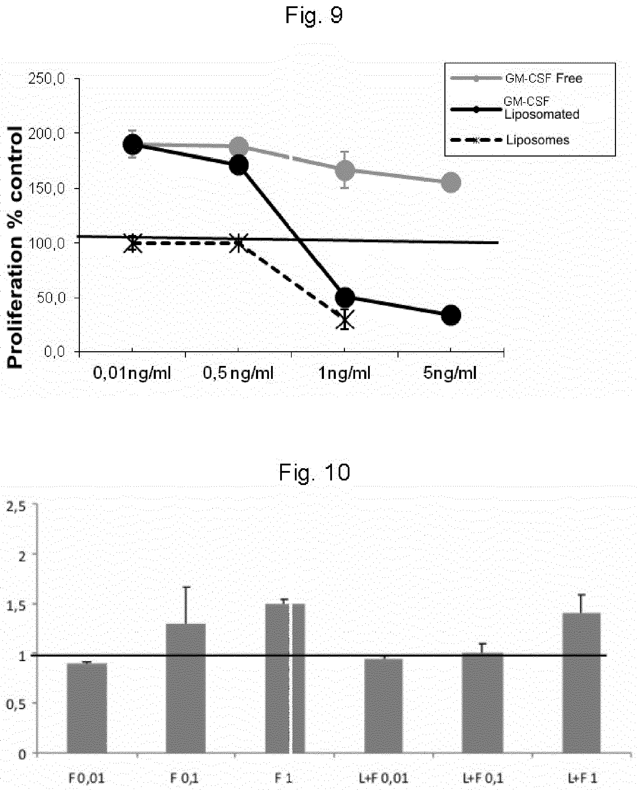

In Figure 8, a slight increase in the repair velocity of the wound is observed in human keratinocytes with respect to the control cells since at 24 hours of the treatment the wound had closed 13% more quickly than the control and at 48 hours it was already completely closed. However, it is observed that the liposomated factor makes it difficult to close the wound, it being closed more slowly than in the control cells. In addition, in Figure 9 it is observed that in human fibroblasts the cells treated with free factor show greater repair velocity of the wound, 20% more quickly with respect to the control. However, like the other cases, the liposomated factor makes it difficult to close the wound, it being closed more slowly than in the control cells.

It should be highlighted that the results obtained with the migration assays are similar to those obtained in the cell proliferation assays and therefore they corroborate them.

3. INDUCTION OF ELASTIN SYNTHESIS BY MEANS OF MEASURING TRANSCRIPTS OF THE GENE OF THE ELASTIN BY QUANTITATIVE PCR

3.1 INDUCTION OF ELASTIC SYNTHESIS BY HGH

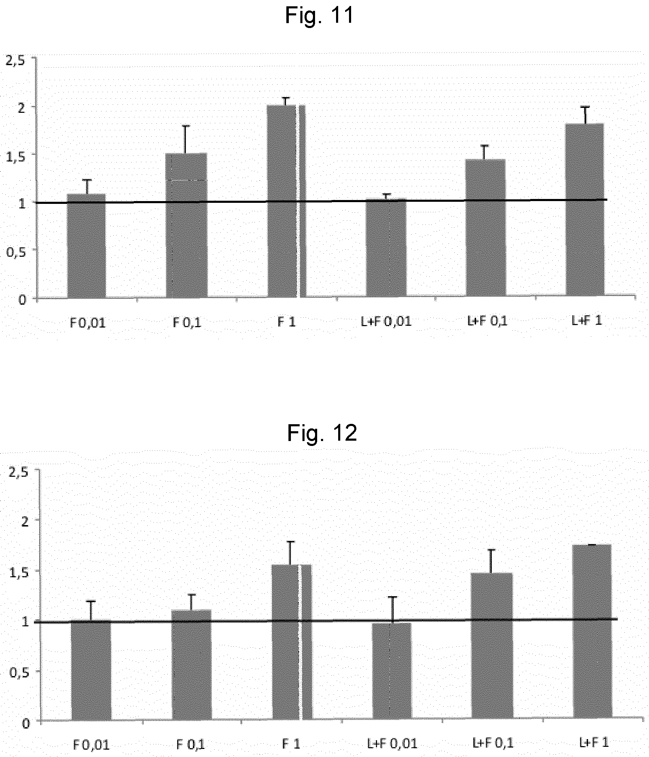

In Figures 10 and 11, an increase in the synthesis of elastin is observed in the cells treated both with the free factor and with the liposomated factor with respect to the control cells (indicated by the horizontal line = value 1), both in fibroblasts and in keratinocytes. This increase is dose dependent, increasing as the concentration of the factor is increased.

3.2 INDUCTION OF ELASTIN SYNTHESIS BY GM-CSF

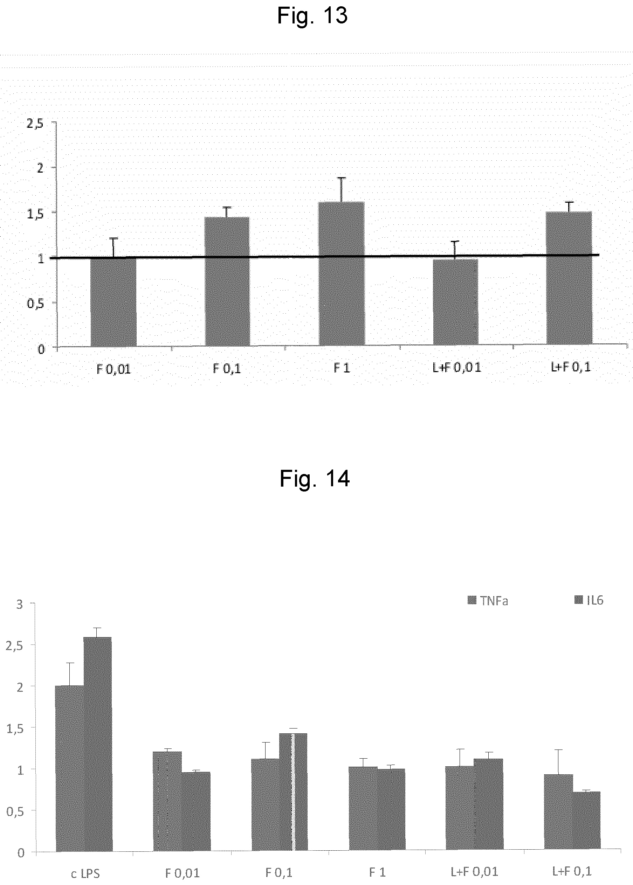

In Figures 12 and 13, an increase in the synthesis of elastin is observed in the cells treated both with the free factor and with the liposomated factor with respect to the control cells (indicated by the horizontal line = value 1), both in fibroblasts and in keratinocytes. This increase is dose dependent, increasing as the concentration of the factor is increased.

4. ANALYSIS OF THE ANTI-INFLAMMATORY EFFECT BY MEANS OF MEASURING TRANSCRIPTS OF THE ANTI-INFLAMMATORY GENES IL6 AND TNFa BY QUANTITATIVE PCR

In this assay on the cells, a mild inflammation was induced on them with LPS prior to the treatment. To this end, medium with 200 ng/ml LPS was added to them, for three hours, it was removed and the medium with the factors was added.

4.1 ANALYSIS OF THE ANTI-INFLAMMATORY EFFECT INDUCED BY HGH

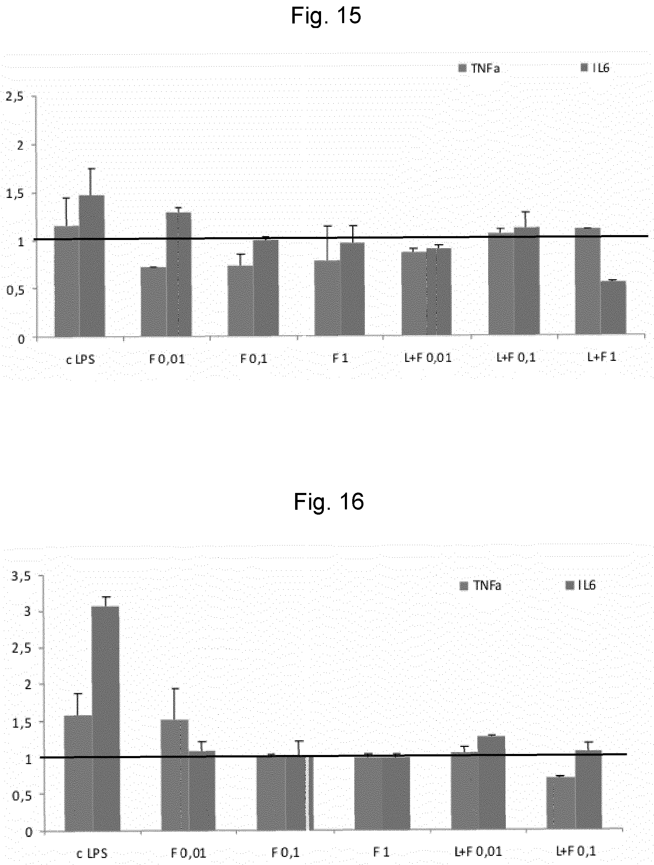

In the Figures 14 and 15, a decrease of the inflammation is observed, that is to say, the transcript levels of TNFa and IL6 in the cells treated with the free factor or with the liposomated factor after prior inflammation with LPS (LPS c bars) having been induced in them. These results indicate the anti-inflammatory effect of the HGH factor, both free and liposomated. This decrease is dose dependent, increasing as the concentration of the factor is increased.

4.2 ANALYSIS OF THE ANTI-INFLAMMATORY EFFECT INDUCED BY GM-CSF

In Figure 16, a decrease of the inflammation is observed, that is to say, the transcript levels of TNFa and IL6 in the cells treated with the free factor or with the liposomated factor after prior inflammation with LPS (LPS c bars) having been induced in them. These results indicate the anti-inflammatory effect of the GM-CSF factor, both free and liposomated. This decrease is dose dependent, increasing as the concentration of the factor is increased.

With the nature of the present invention sufficiently described, all that remains to be added is that said invention may undergo certain variations in terms of components and percentages, provided that said alterations stay within the scope of the invention according to the appended claims.

**Claims**

1. A cosmetic product with liposomated growth factors that, including a mixture of liposomes which incorporate in their interior antioxidants such as ergothioneine, quercetin and pterostilbene, a mixture of liposomes of anti-wrinkle peptides and an extract of *Malus domestica* stem cells not included in liposomes, which comprises a mixture with the following percentages of liposomes of the following growth factors of plant origin:

- HGH liposomes (human growth hormone) *Nicotiana benthamiana* Sh-Polypeptide-7 of 4 to 10%.
- GM-CSF liposomes (Granulocyte Macrophage Colony-Stimulating Factor) *Nicotiana benthamiana* Sh-Polypeptide-45 of 2 to 7%.
- TGFb2 liposomes (Tumor growth factor beta 2) *Nicotiana benthamiana* Hexapeptide-40 Sh-Polypeptide-76 of 2 to 10%.

2. The cosmetic product with liposomated growth factors according to claim 1, **characterized in that** the liposomes encapsulate each one of the growth factors, antioxidants and anti-wrinkle peptides separately.

3. The cosmetic product with liposomated growth factors according to claim 1, **characterized in that** the growth factors included in the liposomes have a purity greater than 95%.

4. The cosmetic product with liposomated growth factors according to claim 1, **characterized in that** the percentages of liposomes with antioxidants in the interior is of 1 to 3%.

5. The cosmetic product with liposomated growth factors according to claim 1, **characterized in that** the anti-wrinkle peptides are palmitoyl tripeptide-3, caprooyl tetrapeptide-3 and *centella asiatica* extract.

6. The cosmetic product with liposomated growth factors according to claim 1 and 5, **characterized in that** the percentages of liposomes with the three anti-wrinkle peptides palmitoyl tripeptide-3, caprooyl tetrapeptide-3 and *centella asiatica* extract in the interior is up to 6%.

7. The cosmetic product with liposomated growth factors according to claim 1, **characterized in that** the percentage of *Malus domestica* stem cell extract is of 0.1 to 1.5%.


**Patentansprüche**

1. Ein kosmetisches produkt mit liposomierten wachstumsfaktoren, das eine mischung aus liposomen mit antioxidantien wie ergothionein, quercetin und pterostilben, eine mischung aus liposomen mit antifaltenpeptiden und einen extrakt aus *Malus* domestica-stammzellen (nicht in liposomen eingeschlossen) enthält. Das produkt enthält die folgenden prozentsätze an liposomen der folgenden wachstumsfaktoren pflanzlichen ursprungs:

- HGH-Liposomen (menschliches wachstumshormon) *Nicotiana benthamiana* Sh-Polypeptid-7 von 4 bis 10 %.
- GM-CSF-Liposomen (granulozyten-makrophagen-kolonie-stimulierender faktor) *Nicotiana benthamiana* Sh-Polypeptid-45 von 2 bis 7 %.
- TGFb2-Liposomen (turner-wachstumsfaktor Beta 2) *Nicotiana benthamiana* Hexapeptid-40 Sh-Polypeptid-76 von 2 bis 10 %.

2. Kosmetisches produkt mit liposomierten wachstumsfaktoren nach anspruch 1, **dadurch gekennzeichnet, dass** die liposomen jeweils einen der wachstumsfaktoren, antioxidantien und anti-falten-peptide separat einkapseln.

3. Kosmetisches produkt mit liposomierten wachstumsfaktoren nach anspruch 1, **dadurch gekennzeichnet, dass** die in den liposomen enthaltenen Wachstumsfaktoren eine reinheit von über 95 % aufweisen.

4. Kosmetisches produkt mit liposomierten wachstumsfaktoren nach anspruch 1, **dadurch gekennzeichnet, dass** der anteil der liposomen mit antioxidantien im Inneren 1 bis 3 % beträgt.

5. Kosmetisches produkt mit liposomierten wachstumsfaktoren nach anspruch 1, **dadurch gekennzeichnet, dass** die anti-falten-peptide palmitoyltripeptid-3, caprooyltetrapeptid-3 und Centella-asiatica-extrakt sind.

6. Das kosmetische produkt mit liposomierten wachstumsfaktoren nach anspruch 1 und 5, **gekennzeichnet durch** einen anteil von liposomen mit den drei anti-falten-peptiden palmitoyltripeptid-3, caprooyltetrapeptid-3 und *Centella-asiatica*-extrakt im Inneren von bis zu 6 %.

7. Das kosmetische produkt mit liposomierten wachstumsfaktoren nach anspruch 1, **gekennzeichnet durch** einen anteil von *Malus-domestika*-stammzellextrakt von 0,1 bis 1,5 %.

**Revendications**

1. Un produit cosmétique contenant des facteurs de croissance liposomés, comprenant un mélange de liposomes incorporant des antioxydants tels que l'ergothionéine, la quercétine et le ptérostilbène, un mélange de liposomes de peptides antirides et un extrait de cellules souches de *Malus domestica* non inclus dans les liposomes, et comprenant un mélange contenant les pourcentages suivants de liposomes des facteurs de croissance d'origine végétale suivants :

   - Liposomes d'hormone de croissance humaine (HGH) *Nicotiana benthamiana* Sh Polypeptide-7 : 4 à 10 %.
   - Liposomes de GM-CSF (facteur de stimulation des colonies de granulocytes et de macrophages) *Nicotiana benthamiana* Sh Polypeptide-45 : 2 à 7 %.
   - Liposomes de TGFb2 (facteur de croissance Turner bêta-2) *Nicotiana benthamiana* Hexapeptide-40 Sh Polypeptide-76 : 2 à 10 %.

2. Produit cosmétique contenant des facteurs de croissance liposomés selon la revendication 1, **caractérisé en ce que** les liposomes encapsulent séparément les facteurs de croissance, les antioxydants et les peptides antirides.

3. Produit cosmétique contenant des facteurs de croissance liposomés selon la revendication 1, **caractérisé en ce que** les facteurs de croissance contenus dans les liposomes présentent une pureté supérieure à 95 %.

4. Produit cosmétique contenant des facteurs de croissance liposomés selon la revendication 1, **caractérisé en ce que** le pourcentage de liposomes contenant des antioxydants est compris entre 1 et 3 %.

5. Produit cosmétique contenant des facteurs de croissance liposomés selon la revendication 1, **caractérisé en ce que** les peptides antirides sont le palmitoyl tripeptide-3, le caprooyl tétrapeptide-3 et l'extrait de *Centella asiatica.*

6. Produit cosmétique contenant des facteurs de croissance liposomés selon les revendications 1 et 5, **caractérisé en ce que** le pourcentage de liposomes contenant les trois peptides antirides palmitoyl tripeptide-3, caprooyl tétrapeptide-3 et l'extrait de *Centella asiatica* peut atteindre 6 %.

7. Produit cosmétique contenant des facteurs de croissance liposomés selon la revendication 1, **caractérisé en ce que** le pourcentage d'extrait de cellules souches de *Malus domestica* est compris entre 0,1 et 1,5 %.

## Fig. 1

## Fig. 2

## Fig. 3

## Fig. 4

## Fig. 5

## Fig. 6

## Fig. 7

## Fig. 8

## Fig. 9

## Fig. 10

## Fig. 11

## Fig. 12

## Fig. 13

## Fig. 14

## Fig. 15

## Fig. 16

**EP 3 162 357 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009026949 A
- EP 1941901 A
- US 2004265268 A
- WO 2010001417 A